(19) Europäisches Patentamt
European Patent Office
Office européen des brevets

(11) **EP 2 005 983 A2**

(12) **EUROPEAN PATENT APPLICATION**
published in accordance with Art. 153(4) EPC

(43) Date of publication:
**24.12.2008 Bulletin 2008/52**

(51) Int Cl.:
*A61M 1/18* (2006.01)   *B01D 69/08* (2006.01)
*B01D 69/12* (2006.01)   *B01D 71/12* (2006.01)

(21) Application number: **07715274.2**

(22) Date of filing: **07.03.2007**

(86) International application number:
**PCT/JP2007/054383**

(87) International publication number:
**WO 2007/102528 (13.09.2007 Gazette 2007/37)**

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **09.03.2006   JP 2006064170**

(71) Applicant: **Toyo Boseki Kabushiki Kaisha
Osaka-shi, Osaka 530-8230 (JP)**

(72) Inventors:
• **MONDEN, Noriko,
c/o TOYO BOSEKI KABUSHIKI KAISHA
Ohtsu-shi Shiga 5200292 (JP)**

• **SAGARA, Takahito,
c/o TOYO BOSEKI KABUSHIKI KAISHA
Ohtsu-shi Shiga 5200292 (JP)**
• **YAMAMOTO, Isamu,
c/o TOYO BOSEKI KABUSHIKI KAISHA
Ohtsu-shi Shiga 5200292 (JP)**

(74) Representative: **Vossius & Partner
Siebertstrasse 4
81675 München (DE)**

(54) **HOLLOW FIBER MEMBRANE WITH EXCELLENT PERFORMANCE STABILITY AND BLOOD PURIFIER AND METHOD FOR PRODUCING HOLLOW FIBER MEMBRANE**

(57) **Purpose:** To provide a blood purifier having a high water permeability, for use in treatment of chronic renal failure, which is not variable in performance during the treatment, independently of a patient's body condition.

**Solution:** The present invention provides a hollow fiber membrane excellent in performance stability, which has an average thickness of from 10 to 50 $\mu$m and an average pore radius of from 150 to 300 Å, and which shows a pure water permeability of 150 to 1,500 mL/m$^2$/hr./mmHg at 37°C, **characterized in that** the ratio of the overall mass transfer coefficient (Ko$\beta$2) of a blood plasma solution of $\beta$2-microgloburin to the overall mass transfer coefficient (Komyo) of an aqueous myogloburin solution (i.e., Ko$\beta$2/Komyo) is from 0.7 to 1.0.

**Description**

TECHINICAL FIELD

The present invention relates to hollow fiber membranes having high water permeability and high performance stability and blood purifiers.

BACKGROUND OF THE INVENTION

In the hemocathartic treatments for renal failure, etc., blood purifiers such as hemodialyers, blood filters, hemodialytic filters, etc. are widely used to remove urine toxic substances and waste products in blood. Such blood purifiers comprise, as separators, dialytic membranes or ultrafiltration membranes which are manufactured from natural materials such as cellulose or derivatives thereof (e.g., cellulose diacetate, cellulose triacetate, etc.) and synthesized polymers such as polysulfone, polymethyl methacrylate, polyacrylonitrile, etc. Particularly, blood purifiers using hollow fiber membranes as separators are highly important in the field of blood purifiers because of their advantage such as reduction of the amount of extracorporeal circulated blood, high efficiency of removing blood substances, high productivity of assembling blood purifiers.

Blood purifiers comprising hollow fiber membranes are mainly used to remove low molecular weight substances such as urea and creatinine from blood as follows: blood is usually allowed to flow into the hollow portion of a hollow fiber membrane, and a dialyzing fluid is allowed to flow in the opposite direction outside the hollow fiber membrane so that the blood diffuses to the dialyzing fluid to transfer the substances so that the above-described low molecular weight substances are removed from the blood. Complications due to dialyses have raised public issues with increase in the number of patients who require dialyses over long periods of time. Recently, objective substances to be removed by dialyses include not only the low molecular weight substances such as urea and creatinine but also substances having medium molecular weights of several thousands and substance having high molecular weights of from 10,000 to 20,000. Under such a circumstance, there arises a demand for bloom-purifying membranes capable of removing these substances. Especially, β2 microglobulin having a molecular weight of 11,700 is known to be a causative substance of carpal tunnel syndrome and thus is designated as a target substance to be removed. Membranes for use in removal of such high molecular weight substances are called high performance membranes, which are improved in high molecular weight substance-removing efficiency by increasing the pore diameters or the number of the pores or the porosity of the membranes, or decreasing the thickness of the membranes, as compared with the conventional dialyzing membranes.

However, the above-described high performance membranes, undesirably, also permit leakage of useful blood proteins, i.e., albumin (having a molecular weight of 66,000) therefrom, in spite of their excellent β2 microglobulin-removing performance. To compensate this defect, it is considered that the fractional properties of the membranes should be sharpened. There is disclosed a process for manufacturing hollow fiber membranes having sharp fractional properties (cf. Patent Publication 1), wherein the hollow fiber membranes have two-layer or multilayer structures and have minute layers at least on the interiors thereof, so that the rate of decrease in sieving coefficient for medium and large molecules in blood plasma is lowered to a predetermined value or less, in comparison with a sieving coefficient in an aqueous solution. By doing so, permeation of the medium and large molecules by filtration can be decreased without lowering permeation of the same by diffusion, which makes it possible to manufacture membranes having sharp fractional properties.

There are also disclosed inventions intended to provide membranes having sharp fractional properties (cf. Patent Publications 2 and 3), wherein, in the manufacturing process of hollow fiber membranes, the compositions of raw materials are changed to control the solidifying rates of spinning dopes, to thereby narrow the widths of the pore size distributions and to cause membranes to have uniform structures.

There is further disclosed a method for obtaining sharp fractional properties (cf. Patent Publication 4), wherein a hollow fiber membrane is caused to have a coarse structure to thereby control a ratio of a porosity of the membrane to a sieving coefficient of the membrane for albumin and myoglobin within a predetermined range so as to obtain sharp fractional properties.

In these inventions, the hollow fiber membranes are caused to have minute layers on their inner surfaces to thereby prevent clogging of the hollow fiber membranes due to the adsorption of blood proteins thereonto, so that sharp fractional properties and maintenance of such properties can be attained.

There is further disclosed improvement of smoothness of the inner surfaces of membranes, and this technique is described to be effective to prevent clogging of the membranes and to improve the fractional properties and time stability of the membranes. As the means for improving the smoothness of the inner surfaces of the membranes, there is employed dry and wet spinning, using a gas as a hollow portion-forming material in the step of spinning a hollow fiber membrane (cf. Patent Publication 5).

There is disclosed a hollow fiber membrane which has a high separation efficiency because of its specific plasma protein adsorption style onto its inner surface, found when the plasma comes into contact with the membrane (cf. Patent Publication 6). This is because the amorphous region and the crystal region which constitute the pore sizes and the membrane of the hollow fiber membrane can take proper balance.

There is further disclosed a hollow fiber membrane having an active layer whose structure has a specific pore size in accordance with an objective substance to be removed and has a specific number of pores, so that substances such as $\beta2$ microglobulin, etc. can be efficiently removed while inhibiting permeation of proteins (cf. Patent Publication 7).

There is disclosed a hollow fiber membrane having a smooth surface in order to improve the time stability thereof and having a decreased inner pore size in order to improve the flow rate of blood (cf. Patent Publication 8).

As described above, minute inner surfaces of membranes, improved smoothness thereof and control of pore sizes thereof within a predetermined range, according to objective substances to be removed are reported to be effective to sharpen their fractional properties, to suppress adsorption of blood proteins onto the membranes and to prevent time change of the membranes. However, even the membranes having such characteristic structures are hard to obtain performance stability in clinical treatments. For example, the use of the hollow fiber membranes improved in smoothness of their inner surfaces is expected to be effective to suppress adsorption of blood proteins thereonto. However, the blood conditions of patients who undergo blood purification therapy differ from one another; or there is difference in treating effect or removing performance of the hollow fiber membranes, among each of patents or in the same patient, depending on the body conditions of the patients who are undergoing the treatments. Therefore, reproducibility of the treating effect in a restricted meaning is not always high. The same evaluation is also derived from the use of the membranes having a specifies pore size within the predetermined range in accordance with the objective substance to be removed. A hollow fiber membrane which is designed taking, out of consideration, change of the apparent pore size of the membrane during a treatment, is not likely to obtain intended performance because of change in the condition of the membrane surfaces due to contact with blood. The same evaluation is also found in time change of performance of the blood purifier: the time stability of the blood purifier tends to change depending on the condition of a patients blood, which leads to a disadvantage in reproducibility of the treating effect. These phenomena arise problems also in manufacturing of blood purifiers which have different membrane surface areas, respectively, despite the use of the same hollow fiber membranes. Therefore, it is needed to repeat lots of blood tests for development of blood purifiers. The present inventors have extensively studied hollow fiber membranes for use in blood purifiers in order to solve the above-described problems. As a result, they have succeeded in manufacturing of hollow fiber membranes which show performance lessened in blood dependency and which are excellent in performance stability during clinical treatments and thus are suitable for blood purifiers, by controlling a ratio between the performance of the hollow fiber membranes in the water system and the performance thereof in the blood system to be constant.

Patent Publication 1: JP-A-10-127763/1998
Patent Publication 2: JP-A-10-165774/1998
Patent Publication 3: JP-A-2000-153134/2000
Patent Publication 4: JP-A-10-216489/1998
Patent Publication 5: JP-A-10-108907/1998
Patent Publication 6: JP-A-2000-300973/2000
Patent Publication 7: JP-B-6-42905/1994
Patent Publication 8: JP-A-8-970/1996

BRIEF DESCRIPTION OF DRAWINGS

Fig. 1 is a schematic diagram illustrating an example of the structure of the section of a hollow fiber membrane according to the present invention.
Fig. 2 is a graph showing a general tendency of a relationship between Ko$\beta$2/Komyo and a retention.
Fig. 3 is a graph showing a general tendency of a relationship between a pore volume porosity and Ko$\beta$2/Komyo.

DESCRIPTION OF REFERENCE NUMERALS

1 = a minute layer on a membrane inner surface
2 = a membrane outer surface layer
3 = a membrane support layer

4 = β2-microglobulin
5 = albumin
6 = a protective layer

DISCLOSURE OF THE INVENTION

PROBLEM TO BE SOLVE BY TEH INVENTION

The present invention is intended to provide a blood purifier which has a high water permeability and shows excellent performance stability in its blood system.

MEANS FOR SOLVING THE PROBLEM

The present invention is accomplished as a result of the present inventors' intensive studies for solving the above-described problems. That is, the present invention provides the following.

(1) A hollow fiber membrane excellent in performance stability, which has an average membrane thickness of from 10 to 50 μm and an average pore radius of from 150 to 300 angstrom, **characterized in that** the hollow fiber membrane shows a pure water permeability of from 150 to 1,500 mL/m$^2$/hr./mmHg at 37°C, and in that a ratio of an overall mass transfer coefficient of a plasma solution of β2-microglobulin (Koβ2) to an overall mass transfer coefficient of an aqueous myoglobin solution (Komyo) (Koβ2/Komyo) is from 0.7 to 1.0.
(2) A hollow fiber membrane excellent in performance stability, defined in the item (1), wherein the pore volume porosity is from 10 to 50%.
(3) A hollow fiber membrane excellent in performance stability, defined in the item (1) or (2), wherein the hollow fiber membrane has minute layers on the inner and outer surfaces thereof, and wherein an intermediate layer between each of the minute layers is a support layer having substantially no void.
(4) A blood purifier assembled using a hollow fiber membrane defined in any of the items (1) to (3), which is hard to clog and thus is excellent in performance stability, wherein a myoglobin clearance measured after a blood plasma has been circulated on the blood passage side of the blood purifier for one hour is 60% or more of a myoglobin clearance measured before the circulation of the blood plasma.
(5) A blood purifier excellent in performance stability, defined in the item (4), wherein a variability of the β2-microglobulin clearance measured after the circulation of the blood plasma for one hour is 8% or less.
(6) A process for manufacturing a hollow fiber membrane defined in any of the items (1) to (3), by a dry and wet spinning method, characterized in that a spinning dope is discharged from a nozzle to form a semi-solid filament hollow inside, which is then immersed in a solidifying bath to be solidified to form a hollow fiber membrane, which is successively washed in a water washing tank, while a washing liquid and the hollow fiber membrane are being fed in the same direction.

EFFECT OF THE INVENTION

A blood purifier according to the present invention has a high water permeability and is stable in performance in its blood system. Therefore, the blood purifier has an advantage in that its treating effect is expected to have high reproducibility independently of a patient's blood condition.

BEST MODES FOR CARRYING OUT THE INVENTION

Hereinafter, the present invention will be described in detail.
The present inventors have examined the manufacturing process of hollow fiber membrane for use in blood purifiers and the performance thereof in order to solve the above-described problems. As described above, hollow fiber membranes aiming at high water permeability already have been developed by increasing the pore diameters of the membranes to thereby increase the pore portions of the entire membranes or by decreasing the membrane thickness. Such hollow fiber membranes developed taken into account only high water permeability are more likely to clog due to adsorption of blood proteins onto the surfaces thereof during hemodialyses and hemodialytic filtration, and thus tend to degrade in dialyzing efficiency and filtering efficiency with time. Membranes liable to clog show large variability in transmembrane pressure, and the amounts of protein leaked therefrom largely vary with time. Therefore, the performance of the hollow fiber membranes varies depending on patients' blood conditions during clinical treatments.
In the meantime, hollow fiber membranes increased in pore portions per the entire membranes or hollow fiber membranes decreased in thickness become weaker in strength than the conventional hollow fiber membranes. This drawback becomes serious in the course of manufacturing of the same membranes or in the course of transportation of the same membranes. To obtain hollow fiber membranes having performance reproducibility independently of the conditions of blood to contact, it is important to keep constant the influences of proteins on the performance of the membranes during periods immediately after the start of blood circulation until the completion of the blood circulation. The present inventors have found that a ratio of the performance in the water system and the performance in the blood system and the retention of the performance in the water system found after the contact with blood are effective as indexes for evaluating this property. To satisfy these indexes and to obtain hollow fiber membranes having sufficient strength without any trouble in handling ease, the present inventors have found that there is a close relationship between a gelation rate in a membrane-manuafacturing process and a tension applied to a hollow fiber membrane being formed during a spinning step. The present invention is accomplished based on these findings.

In the present invention, the pure water permeability of the hollow fiber membrane at 37°C is preferably from 150 mL/m$^2$/hr./mmHg inclusive to 1,500 mL/m$^2$/hr./mmHg inclusive. When the water permeability is lower than 150 mL/m$^2$/hr./mmHg, a high water permeability aimed at in the present invention is not attained, and generally, such a hollow fiber membrane is also low in permeability to a medium molecular weight substance in the blood system. When the permeability is too high, the pore diameter of such a hollow fiber membrane becomes larger, which is likely to lead to a larger amount of protein leaked from the membrane. Accordingly, the water permeability of the hollow fiber membrane is more preferably from 150 mL/m$^2$/hr./mmHg inclusive to 1,200 mL/m$^2$/hr./mmHg inclusive, still more preferably from 150 mL/m$^2$/hr./mmHg inclusive to 1,000 mLL/m$^2$/hr./mmHg inclusive.

In the present invention, the average thickness of the hollow fiber membrane is preferably from 10 $\mu$m inclusive to 50 $\mu$m inclusive. When the average thickness of the hollow fiber membrane is too large, permeability to a medium or high molecular weight substance is likely to be insufficient, even though high water permeability can be ensured. Another problem arises from the viewpoint of designing: that is, a blood purifier assembled using the hollow fiber membranes having such a large thickness, undesirably becomes larger in its dimensions when the membrane area is increased. The thinner the thickness of the hollow fiber membrane, the more preferable it is, because a thinner membrane becomes higher in substance permeability. The average thickness of the hollow fiber membrane is more preferably 45 $\mu$m or less, still more preferably 40 $\mu$m or less. When the average thickness of the hollow fiber membrane is too small, a blood purifier comprising such a hollow fiber membrane is hard to maintain the minimum membrane strength necessary therefor. Accordingly, the average thickness of the hollow fiber membrane is more preferably 12 $\mu$m or more, still more preferably 14 $\mu$m or more. The average thickness of the hollow fiber membrane herein referred to means an average value calculated from the thickness of five hollow fiber membranes sampled at random, provided that a difference between the average value and each of the values of the thickness of the hollow fiber membranes should not exceed 20% of the average value.

The inner diameter of the hollow fiber membrane is preferably from 100 to 300 $\mu$m. When the inner diameter is too small, a pressure loss of a fluid passing through the hollow portion of the hollow fiber membrane becomes larger, which may lead to hemolysis. When the inner diameter is too large, the shear rate of blood passing through the hollow portion of the hollow fiber membrane becomes smaller, with the result that proteins in the blood tend to accumulate on the inner surface of the membrane with time. The inner diameter of the hollow fiber membrane suitable to keep appropriate pressure loss or shear rate of the blood passing through the hollow portion of the hollow fiber membrane is from 150 to 250 $\mu$m.

In the present invention, the measurement of an overall mass transfer coefficient is conducted according to the method regulated by the Japanese Society for Dialysis Therapy. This is specifically conducted as follows.

(1) Overall Mass Transfer Coefficient of

Aqueous Myoglobin Solution (Komyo)

In a blood purifier [having a membrane area (A') of 15,000 cm$^2$ based on the inner diameter of a hollow fiber membrane] primed and wetted with a physiological salt solution, a dialyzing fluid which contains 0.01% of myoglobin (manufactured by Kishida Chemical Co., Ltd.) is allowed to flow on the blood side of the membrane at a flow rate (Qbin) of 200 ml/min. as a single path without filtration thereof, while a dialyzing fluid is allowed to flow on the dialyzing fluid side of the membrane at a flow rate (Qd) of 500 ml/min. A clearance (CLmyo, ml/min.) and an overall mass transfer coefficient (Komyo, cm/min.) of the blood purifier are calculated from the myoglobin concentration (Cbin) of the original mylglobin solution, the myoglobin concentration (Cbout) of the solution collected from the outlet of the blood purifier, and the flow rates. The measurement is made at 37°C.

$$CLmyo = (Cbin - Cbout)/Cbin \times Qbin$$

$$Komyo = Qbin/((A' \times (1 - Qbin/Qd)) \times$$
$$LN((1 - CL/Qd)/(1 - CL/Qb))$$

(2) Overall Mass Transfer Coefficient (Ko$\beta$2) of

Plasma Solution of $\beta$2-Microglobulin ($\beta$2-MG)

Blood plasma with a protein concentration of 6 to 7 g/dl is separated from ACD-added bovine blood by centrifugation. Blood plasma for use in a dialyzing test is admixed with heparin sodium (2,000 to 4,000 unit/L) and $\beta$2-microglobulin (a gene-recombination product manufactured by Wako Pure Chemical Industries, Ltd.) in a total amount of about 0.01 mg/dl. Blood plasma for circulation is admixed with heparin sodium alone. At least 2 L of the blood plasma for circulation is prepared per one blood purifier to be measured. The blood plasma for circulation is allowed to flow in a blood purifier (with a membrane area (A') of 15,000 cm$^2$) primed and wetted with a dialyzing fluid at a low rate of 200 ml/min. At this step, the dialyzing fluid side of the blood purifier is filled with a filtrate while the blood plasma being filtered at Qf 15 ml/min. After the passage on the dialyzing fluid side has been filled with the filtrate, the dialyzing fluid side is capped so that the blood plasma is circulated only on the blood side of the blood purifier for one hour. After completion of the circulation, the blood plasma is changed to the blood plasma for dialyzing test. This blood plasma for dialyzing test is allowed to flow as a single path while being filtered, so that Qbin can be 200 ml/min., and Qbout, 185 ml/min., meanwhile a dialyzing fluid is allowed to flow at Qdin of 500 ml/min. After 4 minutes has passed since the start of dialysis, the blood plasma solution Qbout is sampled. A clearance (Cl$\beta$2, ml/min.) and an overall mass transfer coefficient (Ko$\beta$2, cm/min.) are calculated from the $\beta$2-MG concentration (Cbin) of the blood plasma solution and the $\beta$2-MG concentration (Cbout) of the same solution collected from the outlet of the blood purifier and the flow amount (Qbout). All the operations are conducted at 37°C.

$$CL\beta2 = (Cbin \times Qbin - Cbout \times Qbout)/Cbin$$

$$Ko\beta2 = Qbin/((A' \times (1 - Qbin/Qd)) \times$$
$$LN((1 - CL/Qd)/(1 - CL/Qb))$$

A ratio of Ko$\beta$2/Komyo relative to the overall mass transfer coefficients calculated as above is calculated.
In the present invention, Ko$\beta$2/Komyo which is a ratio of the overall mass transfer coefficients in the water system and the blood plasma system is preferably 1 or less. The molecular weight of $\beta$2-MG is about 11,700, while the molecular weight of myoglobin is about 17,000. In general, $\beta$2-MG having a lower molecular weight takes a larger overall mass transfer coefficient (Ko), and thus, it is considered that Ko$\beta$2/Komyo as a ratio of the overall mass transfer coefficients is not smaller than 1. However, the value of Ko$\beta$2 measured in the presence of a blood plasma component is sometimes smaller than the value of Komyo, in spite of the fact that the molecular weight of $\beta$2-MG is smaller than that of myoglobin. Such a behavior is observed with time, and a decrease in the performance of the blood purifier is caused by the blood plasma component's clogging the pores of the hollow fiber membrane, i.e., so-called clogging which arises troubles. In the present invention, it is found that a reversible protein layer rapidly formed on the surface of the hollow fiber membrane acts as a protective layer which suppresses a decrease or variation in the performance of the membrane due to clogging. This is described in detail: when a reversible protective layer or the like, which is not formed in the water system, is formed on the inner surface of the hollow fiber membrane in the blood plasma system, the protective layer acts as resistance to a mass transfer, so that the overall mass transfer coefficient of $\beta$2-MG having a lower molecular weight (a Ko$\beta$2 value (the blood plasma system)) is considered to become smaller than the overall mass transfer coefficient of the myoglobin having a higher molecular weight (a Komyo value the water system)). However, the protective layer herein formed is considered to be formed immediately after the blood plasma component contacts the inner surface of the hollow fiber membrane, because time change in the amount of protein leaked from the blood purifier of the present invention is small. Thus, the protein layer acts as the protective layer formed on the inner surface of the hollow fiber membrane to thereby prevent time change of the performance of the hollow fiber membrane or clogging of the hollow fiber membrane. For the above-described reasons, the blood purifier having the above-described characteristics is found to be higher in reproducibility of the blood performance.

In the hollow fiber membrane of the present invention, Koβ2/Komyo as a ratio of the overall mass transfer coefficients of the water system and the blood plasma system is preferably 1 or less, more preferably 0.98 or less, still more preferably 0.95 or less. When this ratio exceeds 1, the effect of the protective layer due to the blood plasma component is likely to be insufficient, so that a time change in the blood performance is observed or the reproducibility of exhibition of the performance may be poor. In general, hemodialytic treatment is continued for about 3 to about 5 hours. In such a treatment, a treating effect firstly expected sometimes can not be obtained, because of a larger difference between the initial performance of the hollow fiber membrane and the performance thereof after completion of the treatment or because of variability of the degree of time change of the performance of the hollow fiber membrane depending on a patient's blood condition. The lower limit of Koβ2/Komyo as the ratio of the overall mass transfer coefficients in the water system and the blood plasma system is preferably 0.7 or more, more preferably 0.8 or more.

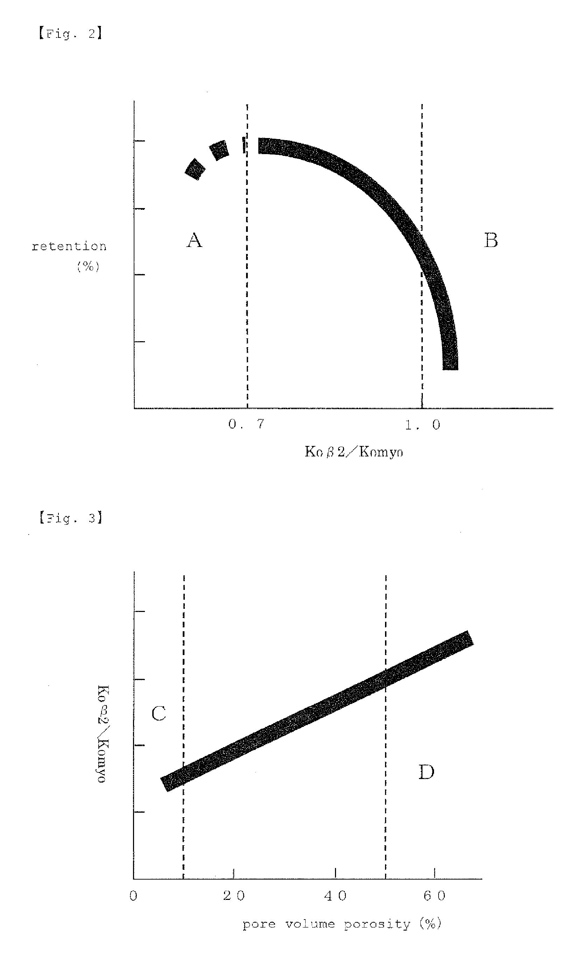

As shown in Fig. 2, when Koβ2/Komyo is less than 0.7, a protein layer formed after the blood component contacts the hollow fiber membrane acts as a resistant layer rather than a protective layer. In this case, time change and variation in the performance of the hollow fiber membrane may be suppressed, however, the performance of the hollow fiber membrane may not be sufficiently exhibited during a clinical treatment (see the region A).

On the other hand, when Koβ2/Komyo exceeds 1, there is no estrangement between the blood system and the water system, and thus, such a hollow fiber membrane appears to be an ideal membrane. However, blood plasma protein does not act as a protective layer, and the surface of the hollow fiber membrane tends to clog with time, so that the performance of the hollow fiber membrane is considered to change with time (see the region B). The retention referred to in the present invention means the performance of the water system found after the hollow fiber membrane contacts the blood plasma (i.e. myoglobin clearance), and the retention indicates the degree of clogging of the membrane when the membrane contacts the blood. While a retention of 100% is impossible because of the resistance of the membrane due to adsorption of proteins thereonto. A retention of 65% or more indicates substantially no degradation of the performance of the membrane due to clogging of the membrane. A retention of less than 60% is supposed to indicate occurrence of irreversible clogging.

In the present invention, the average pore radius of the hollow fiber membrane is preferably from 150 to 300 angstrom, more preferably from 150 to 290 angstrom, still more preferably from 150 to 270 angstrom. When the average pore radius of the hollow fiber membrane is selected within the above-specified range, the influence of objective substances to be removed, such as β2-MG, etc. on the permeating performance is considered to be small, even if there occurs apparent reduction of the pore diameter found after the membrane contacts the blood. When the average pore radius of the hollow fiber membrane is too small, decrease in the performance of the membrane due to the blood components may become remarkable, or time change in the performance thereof may become larger. When the average pore radius of the hollow fiber membrane is too large, the amount of leaked proteins may become too large. In this regard, the average pore radius of the hollow fiber membrane herein referred to is a pore radius which is measured by employing thermal analysis (DSC) described later: for example, the average pore radius is not such one determined from a sieving coefficient of albumin (Stokes' radius of 35 angstrom), but the size of pores in the membrane which is defined and calculated from the state of water in the membrane.

The pore radius determined by DSC is found by using the equations of Laplace and Gibbus-Duhem in combination:

$$r = (2\sigma iw \times Vm \times To \times \cos \theta)/(\Delta T \times \Delta Hm)$$

r: pore radius
σiw: a surface energy (0.01 N/m) between water and ice Vm: molar volume of water
To: melting point of bulk water
θ: contact angle
ΔT: degree of depression of melting point
ΔHm: molar fusion enthalpy,

wherein 0.01 N/m is used as σiw; and AT is a peak top of water which has shown depression of freezing point, observed in DSC measurement, and it is not an average value in strict meaning.
A pore volume porosity is determined from the amount of water which has shown depression of freezing point. As described above, the pore radius is defined and calculated from the state of water in the membrane in DSC measurement.

The retention of the clearance of the water system after the circulation of blood plasma in the present invention is measured as follows. Two blood purifiers of the same type and the same lot (the membrane area based on the inner diameter of the hollow fiber membrane: 1.5 m$^2$) are prepared, and CLmyo of one of the blood purifiers is measured by the above-described method. CLβ2 of the other blood purifier is measured by the above-described method. After that, the blood purifier is washed with water at the same flow rate as in the measurement for 5 minutes. CLmyo of the washed blood purifier is measured, and a ratio of this value to the value of the first blood purifier is calculated. When any decrease in the performance of the blood purifier due to the circulation of blood is not observed, the CLmyo values of the two blood purifiers are equal to each other, and the retention is 100%.

In the present invention, the retention of the clearance of the water system found after the blood plasma has been circulated for one hour is preferably 60% or more, more preferably 64% or more, still more preferably 68% or more. The retention of the water system found after the blood plasma has been circulated for one hour is a parameter useful to evaluate the degree of interaction between the surface of the hollow fiber membrane of the blood purifier and the blood plasma component. If the blood plasma component infiltrates the hollow portion of the hollow fiber membrane and causes clogging which can not be easily eliminated by conventional blood flow, the retention is considered to extremely lower. In other words, when the retention of the clearance of the water system found after the blood plasma has been circulated for one hour is less than 60%, the hollow fiber membrane is considered to be clogged by the blood plasma component, and this is disadvantageous to maintain the performance of the membrane.

In the present invention, the porosity of the hollow fiber membrane is preferably 70% or more, and the yield strength is preferably 8 g/filament or more. The porosity of the hollow fiber membrane is more preferably 72% or more, and the yield strength is more preferably 10 g/filament. The percentage of hole of the hollow fiber membrane is still more preferably 74% or more, and the yield strength is still more preferably 12 g/filament. Generally, the porosity of the hollow fiber membrane has correlation with the water permeability of the hollow fiber membrane, and thus, to obtain a higher water permeability, the porosity of the hollow fiber membrane is increased. When the porosity of the hollow fiber membrane is too low, a higher water permeability aimed at in the present invention may not be obtained. On the other hand, too high a percentage of void or too low a yield strength tends to lower the strength of the hollow fiber membrane, which is likely to lead to poor handling ease or is likely to cause troubles in the step of assembling a module using such a hollow fiber membrane. Therefore, the porosity of the hollow fiber membrane is preferably 90% or less, more preferably 85% or less, still more preferably 80% or less.

Examples of a material for the hollow fiber membrane of the present invention include cellulose-based polymers such as regenerated cellulose, cellulose acetate and cellulose triacetate; polysulfone-based polymers such as polysulfone and polyethersulfone; polyacrylonitrile; polymethyl methacrylate; ethylene-vinyl alcohol copolymers; etc. Among those, cellulose-based polymers and polysulfone-based polymers are preferred because the use thereof facilitates manufacturing of hollow fiber membranes having water permeability of 150 mL/m$^2$/hr./mmHg or more. Particularly preferable cellulose-based polymers are cellulose diacetate and cellulose triacetate, and particularly preferable polysulfone-based polymers are polysulfone and polyethersulfone, because the use thereof makes it easy to decrease the thickness of hollow fiber membranes.

A hollow fiber type blood purifier of the present invention is suitable as a blood purifier for use in treatment of renal failure, such as a hemodialyzer, hemodiafilter, hemofilter or the like. The hollow fiber type blood purifier of the present invention is promising and superior, because difference between each of blood purifiers in one lot, time change in removal performance and water permeability are stable, which enables stable treatments independently of patients' body conditions and symptoms of diseases.

The following conditions for manufacturing a hollow fiber membrane for use in such a blood purifier are preferable. To obtain a hollow fiber membrane having a high water permeability, the polymer concentration of a spinning solution is preferably 26% by mass or less, more preferably 25% by mass or less, which, however, depends on the kind of a polymer to be used. Preferably, the spinning solution is filtered just before the spinning solution is discharged from a nozzle, in order to remove an insoluble component and gel in the spinning solution. The smaller pore size the filter has, the more preferable it is. Specifically, the pore size of the filter is preferably smaller than the thickness of the hollow fiber membrane, more preferably a half or less of the thickness of the hollow fiber membrane. When no filter is used or when the pore size of a filter exceeds the thickness of the hollow fiber membrane, a part of the nozzle slit tends to clog, which may induce occurrence of a hollow fiber membrane with an uneven thickness. Again, when no filter is used or when the pore size of a filter exceeds the thickness of the hollow fiber membrane, an insoluble component or gel in the spinning solution is included in a hollow fiber membrane to cause a partial void or to form a non-uniform texture of the surface of a hollow fiber membrane in the order of several tens μm (i.e., too tensed or partially wrinkled membrane surface). A hollow fiber membrane having a high porosity is lowered in its physical strength due to occurrence of partial void. A hollow fiber membrane having remarkable unevenness in the texture of the surface thereof in the order of several tens μm may activate blood to increase the possibility of causing thrombus and residual blood. Activation of blood is considered to give some influence on the performance stability of the hollow fiber membrane. The filtration of a spinning dope may be repeated several times before the discharge of the spinning dope. This is preferable since the lifetime of a filter can be prolonged.

The spinning dope treated as described above is discharged from a tube-in-orifice type nozzle which has an outer annular portion and an inner hole for discharging a hollow portion-forming material. By decreasing the variance of the slit width of the nozzle (i.e., the width of the annular portion for discharging the spinning dope), unevenness in the thickness of a spun hollow fiber membrane can be decreased. Specifically, a difference between the maximum value and the minimum value of the slit width of the nozzle is preferably 10 $\mu$m or less. The slit width of the nozzle is changed depending on the viscosity of the spinning dope to be used, the thickness of the resultant hollow fiber membrane and the kind of a material for forming a hollow portion. However, a large variance in the slit width of the nozzle induces formation of a hollow fiber membrane with an uneven thickness, which may be torn at its thinner thickness portion or may burst to cause leakage. The use of a hollow fiber membrane with a remarkably uneven thickness makes it difficult to provide a blood purifier having proper strength.

When the spinning dope is discharged, the temperature of the nozzle is preferably set at a temperature lower than the conventional hollow fiber membrane-manufacturing conditions, in order to obtain a sufficient effect in an aeration feeding region in the next step. The temperature of the nozzle is specifically from 50 to 130°C, preferably from 55 to 120°C. When the nozzle temperature is too low, the viscosity of the spinning dope increases to raise a pressure on the nozzle, with the result that the spinning dope can not be stably discharged. Too high a nozzle temperature affects the structure of a hollow fiber membrane formed by phase separation, which is likely to lead to an excessively large pore diameter.

The discharged spinning dope is allowed to pass through the aeration feeding region and is then immersed in a solidifying liquid. Preferably, the aeration feeding region is enclosed by a member capable of shielding from an external air (e.g., a spinning tube) and is kept at a low temperature, specifically 15°C or lower, preferably 13°C or lower, in observation. As a method for controlling the aeration feeding region at a relatively low temperature, a cooling medium is circulated in the spinning tube, or a cooled air is allowed to flow into such a region. Cooling by the cooling medium or the air can be controlled by using a liquid nitrogen or dry ice. The temperature of the aeration feeding region is preferably -20°C or higher in view of operability. It is preferable to uniformly maintain an atmosphere in the aeration feeding region, since such an atmosphere affects the phase separation of the spinning dope. Preferably, the aeration feeding region is covered by fencing, so as not to cause irregularity in the temperature and wind velocity. Irregularity in the atmosphere, temperature and wind velocity of the aeration feeding region causes irregularity in the micro membrane structure, and undesirably, a trouble is caused in exhibition of the performance of the hollow fiber membrane.

To cause no irregularity in the temperature and wind velocity of the aeration feeding region, it is effective to make a device to cause a cooled air to evenly flow by boring holes with proper sizes in the enclosure of the aeration feeding region. While there is no limit in selection of the number of holes bored in the enclosure of the aeration feeding region, it is important to select the number of the holes so as to control the wind to flow over the aeration feeding region and so as not to sway the spun hollow fiber membrane. When the outlet of the nozzle is rapidly cooled, gel tends to form at and around the outlet of the nozzle to clog the nozzle, with the result that the unevenness in the thickness of the hollow fiber membrane becomes significant. To avoid such events, it is one of effective means to insert an heat-insulating material between the nozzle block and the enclosure of the aeration feeding region. There is no limit in selection of the kind of a heat-insulating material, in so far as such a material can isolate heat conduction: for example, ceramics and plastics can be used.

The thickness of the heat-insulating material is preferably from 5 to 20 mm. When this thickness is too thin, insulation of heat is insufficient, and thus, the heat-insulating effect to the nozzle tends to be poor. When this thickness is too thick, the cooling effect in the aeration feeding region is not likely to reflect on the formation of a hollow fiber membrane. By this method, the spinning dope just discharged from the nozzle becomes lower in possibility to close the outlet portion of the nozzle, so that a hollow fiber membrane having high circularity can be stably manufactured. When the nozzle temperature is properly lowered to thereby keep lower the temperature of the aeration feeding region, the gelation rate in the membrane-forming step can be controlled to be constant. When the aeration feeding region is set at a temperature lower than the conventional ones, rapid gelation on the outer surface of a hollow fiber membrane is accelerated, so that the resultant hollow fiber membrane has a three-layer structure wherein the section of the membrane has minute inner and outer layers in comparison with the intermediate portion of the membrane. A hollow fiber membrane having such a three-layer structure is effectively improved in its strength.

The smaller a draft ratio, the better it is. The draft ratio is preferably from 1 to 10, more preferably 8 or less. The draft ratio herein referred to means a ratio of the linear speed of a spinning dope discharged from a nozzle to the take-up speed of the resultant hollow fiber membrane. When the draft ratio is too large, pores are formed in a membrane under a tension, so that the shapes of the pores deform, which is likely to lead to a lower permeability.

A hollow portion-forming material discharged together with the spinning dope from the nozzle gives a significant influence on the formation of the inner surface structure of the hollow fiber membrane. To improve the blood compatibility of the membrane, the structure of the blood-contacting surface of the hollow fiber membrane is important. Stable blood performance features the hollow fiber membrane of the present invention. To achieve such stable blood performance, the hollow fiber membrane is so designed as to have an appropriate protective layer formed of a blood component in the proximity of the inner surface of the hollow fiber membranes. Stability of the blood performance of the hollow fiber membrane means that the hollow fiber membrane is not clogged by a blood component, or that the influence of clogging on the performance of the hollow fiber membrane is, at least, suppressed to be lower. Clogging of the pores of the inner surface of the hollow fiber membrane leads to time change or partial change of the filtering rate, which may induce difference in the amount of leaked protein. This is disadvantageous for stable blood performance which the present invention is intended to achieve.

To manufacture a hollow fiber membrane in which a proper protective layer can be formed of a blood component in the proximity of the inner surface thereof, the composition of a hollow portion-forming material, a nozzle temperature, a draft ratio and a low drawing rate in the spinning step are found to be important. By optimizing these conditions, it is considered that phase separation of the inner surface of a hollow fiber membrane can be controlled so that the degree of unevenness can be adjusted within a proper range.

While the hollow portion-forming material may be selected in accordance with the spinning dope to be used, an inert liquid or gas is preferably used. Specific examples of such a hollow portion-forming material include liquid paraffin, isopropyl myristate, nitrogen, argon, etc. To form a minute layer, an aqueous solution of the solvent for use in the preparation of the spinning dope or water may be used. Each of these hollow portion-forming materials optionally may be admixed with a non-solvent such as glycerin, ethylene glycol, triethylene glycol or polyethylene glycol, or water.

As a means for suppressing the influence of clogging of the pores due to a blood protein on the performance of the hollow fiber membrane, cooling of the aeration feeding region is effective. When the spinning dope discharged from the nozzle is rapidly cooled in the aeration feeding region, a minute layer is formed on the outer surface of the membrane. By forming such a minute layer on the outermost layer of the membrane, it becomes possible to increase a clogging-possible region of the membrane, when the membrane contacts blood to cause clogging. Therefore, the influence of clogging on the performance of the hollow fiber membrane can be suppressed lower.

The gelled membrane, after passing through the aeration feeding region, is allowed to pass through a solidifying bath to be solidified. The solidifying bath is preferably an aqueous solution of the solvent used in the preparation of the spinning dope. When the solidifying bath is water, the gelled membrane is quickly solidified to form a minute layer on the outer surface of the membrane. The rapidly solidified surface of the membrane has a low rate of pore area, however, is hard to control the surface roughness thereof. Preferably, the solidifying bath is a mixture of the solvent and water, because control of a solidifying time and proper adjustment of the surface roughness of the hollow fiber membrane become easy. The solvent concentration of the solidifying bath is preferably 70% by mass or less, more preferably 50% by mass or less. However, the lower limit of the solvent concentration is preferably 1% by mass or more. This is because, when the solvent concentration is 1% by mass or less, control of the concentration during the spinning step is difficult. The temperature of the solidifying bath is preferably from 4 to 50°C, more preferably from 10 to 45°C, in view of control of the solidifying rate. When the hollow fiber membrane is mildly formed in the aeration feeding region and the solidifying bath as described above, the resultant hollow fiber membrane can have a proper number of pores with proper sizes, properly distributed. The solidifying bath optionally may be admixed with additives such as a non-solvent (e.g., glycerin, ethylene glycol, triethylene glycol or polyethylene glycol), an antioxidant, a lubricant, etc.

The hollow fiber membrane which has undergone the solidifying bath is subjected to a washing step to thereby remove unnecessary components such as the solvent. The washing liquid to be used in this step is preferably water, and the temperature of the washing liquid is preferably from 20 to 80°C, within which the washing effect becomes higher. When the temperature of the washing liquid is lower than 20°C, the washing effect is poor. When it is higher than 80°C, heat efficiency is poor; a burden on the hollow fiber membrane is large; and adverse influences are given on the storage stability and performance of the hollow fiber membrane. The hollow fiber membrane is still active even after the solidifying bath step, and the structure, surface condition and the shape of the pores of the hollow fiber membrane are likely to change, when a force is applied thereto from an external in the solidifying bath. Therefore, it is preferable to make such a device that a resistance can not be applied to the hollow fiber membrane being fed in the solidifying bath as much as possible. To remove the unnecessary components such as the solvent and the additives from the hollow fiber membrane, it is preferable to facilitate the renewal of the washing liquid. Conventionally, for example, a hollow fiber membrane is fed while being exposed to a shower of a washing liquid; or a washing efficiency is increased by opposing the feeding of a hollow fiber membrane to the flow of a washing liquid. However, these washing methods have a problem in that the feeding resistance of the hollow fiber membrane becomes larger. Consequently, it is needed to draw the hollow fiber membrane so as to prevent the hollow fiber membrane from loosening or entangling.

The present inventors have extensively studied to satisfy both the requirements, i.e., prevention of deformation of a hollow fiber membrane and improvement of washing efficiency for the hollow fiber membrane. As a result, they have found that it is effective to allow a washing liquid and a hollow fiber membrane to flow in parallel to each other. This is described in detail. For example, there is employed an apparatus in which a washing bath is inclined so that a hollow fiber membrane can flow down along such a slope. Specifically, the inclination of the bath is preferably from 1 to 3°. When the inclination is 3° or more, the flow rate of the washing liquid is too high, and the feeding resistance of the hollow fiber membrane can not be suppressed. When the inclination is less than 1°, the washing liquid tends to remain in the washing bath, and thus, failure in washing of the hollow fiber membrane is likely to occur. When the resistance to the hollow fiber membrane in the washing bath is suppressed as described above, the feeding speed of the hollow fiber membrane at the inlet of the washing bath can be substantially equal to the feeding speed thereof at the outlet of the washing bath. Specifically, the drawing ratio in the washing bath is preferably from 1 to less than 1.2. To improve the washing efficiency, it is preferable to use a multistage washing bath. The number of the stages of the washing bath is needed to be appropriately selected in accordance with the washing efficiency. For example, 3 to 30 stages are sufficient in order to remove a solvent, non-solvent, hydrophilicity-imparting agent, etc., which are to be used in the present invention.

If needed, the hollow fiber membrane which has undergone the washing step is treated with glycerin. For example, a hollow fiber membrane comprising a cellulose-based polymer is allowed to pass through a glycerin bath and is then subjected to a drying step and is then wound up. In this case, the concentration of glycerin is preferably from 30 to 80% by mass. When the glycerin concentration is too low, the hollow fiber membrane is liable to shrink during the drying step, and thus, the storage stability of the hollow fiber membrane tends to degrade. When the glycerin concentration is too high, an excess of glycerin is liable to adhere to the hollow fiber membrane. When a blood purifier is assembled using such a hollow fiber membrane, the end portions of the hollow fiber membrane become poor in adhesiveness. The temperature of the glycerin bath is preferably from 40 to 80°C. When the temperature of the glycerin bath is too low, the viscosity of the aqueous glycerin solution becomes higher, and such an aqueous glycerin solution is not likely to infiltrate the overall pores of the hollow fiber membrane. When the temperature of the glycerin bath is too high, the hollow fiber membrane is likely to be denatured due to heat and deteriorate.

During the entire spinning step, a tension applied to the hollow fiber membrane influences the structure of the hollow fiber membrane, and thus, it is desirable not to draw the hollow fiber membrane as much as possible, in order not to cause a change in the structure of the membrane. This is because the hollow fiber membrane is still active even after the solidifying step, so that the membrane structure, the surface structure of the membrane and the shapes of the pores of the membrane are changed, when an external force is applied to the hollow fiber membrane in the washing bath. Drawing the membrane deforms particularly the shapes of the pores from circle to ellipse, which gives significant influence on the permeability of the membrane. Therefore, the lower a draw ratio, the more desirable it is. Specifically, a ratio between the hollow fiber membrane-feeding rate at the outlet of the solidifying bath and the winding rate thereof at the final stage of the spinning step is preferably from 1 to less than 1.2.

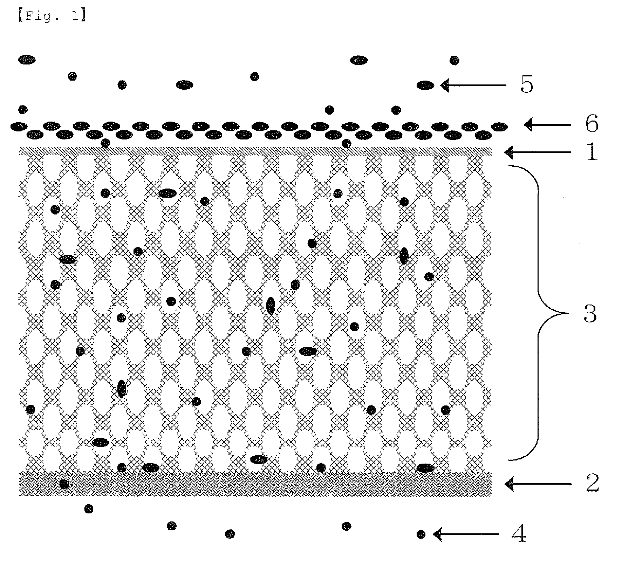

In the structure of the hollow fiber membrane thus treated, the average pore size is from 150 to 300 angstrom, and the proportion of the pores for carrying out separation, i.e., the pore volume porosity, is suppressed to preferably 50% or less; and a mild three-layer structure as shown in Fig. 1 is formed. It is considered that, because of this structure, it becomes possible to form a protective layer in the proximity of the inner surface of the hollow fiber membrane, while preventing infiltration of blood components into the membrane, when the hollow fiber membrane contacts blood. This feature comes from a technical idea different from a sharp cut-off (i.e., a minute layer on the inner surface of a membrane) which the prior art has aimed at. The formed protective layer is considered to be a layer of which the components will be sequentially replaced, but not a layer irreversibly adsorbed onto the membrane. In other words, this phenomenon of a protective layer is not such one that a protein is pushed into the pores of the membrane surface to clog them. For this reason, the hollow fiber membrane of the present invention is free of the problem of the conventional membranes for use in blood purifiers, i.e., the problem of time change or decrease in the performance of the membrane, attributed to clogging of the membrane. Therefore, stability of blood performance featuring the present invention can be obtained.

The reason why the hollow fiber membrane manufactured under the above-described conditions has a feature of Koβ2/Komyo ≤ 1 as a ratio of overall mass transfer coefficients is described below. In comparison with a hollow fiber membrane having similar clearance performance, manufactured by a known manufacturing process, the hollow fiber membrane of the present invention is found to be lower in pore volume porosity. The pore volume porosity means a ratio of the volume of the pores to the volume of the hollow fiber membrane. For example, in case of a hollow fiber membrane comprising a cellulose acetate material, this percentage can be calculated by thermal analysis. The sizes of the pores of different hollow fiber membranes each having similar clearance performance are considered to be similar to each other. However, the pore volume porosity of the hollow fiber membrane manufactured by the process of the present invention shows a smaller value than those of hollow fiber membranes manufactured by the known processes. This means that the hollow fiber membrane of the present invention has a relatively small number of pores in comparison with the known hollow fiber membranes. A lower pore volume porosity comes from the effect produced by cooling the aeration feeding region in the manufacturing process of the hollow fiber membrane of the present invention. In other words, this feature comes from the structure of the hollow fiber membrane of the present invention: a minute layer which has never been formed by any of the conventional spinning methods is formed on the outer surface of the membrane, and additionally, the structure of a whole of the membrane is advantageous to form a protective layer suitable to prevent clogging.

In the present invention, the pore volume porosity of the hollow fiber membrane is preferably from 10 to 50%, more preferably from 20 to 50%, still more preferably from 30 to 48%, far still more preferably from 35 to 45%. Fig. 3 shows a general tendency in the relationship between a pore volume porosity and Koβ2/Komyo. It is found that, as the pore volume porosity increases, the ratio or Koβ2/Komyo tends to increase. Too high a percentage is not only disadvantageous for formation of a protective layer but also disadvantageous in that clogging is liable to occur (see the region D). Too low a percentage leads to an excessively small number of pores, which may result in insufficient β2-MG-removing performance (see the region C).

In the present invention, it is preferable for the hollow fiber membrane to have a structure comprising inner and outer surfaces having minute layers thereon, respectively, and an intermediate portion consisting of a support layer with substantially no void. For example, when the hollow fiber membrane of the present invention is used for a blood purifier, blood is allowed to flow into the hollow portion of the hollow fiber membrane, and a dialyzing liquid is allowed to flow outside the hollow fiber membrane. In this step, the minute layer on the inner surface of the hollow fiber membrane acts to suppress clogging of the pores due to the macromolecular components of the blood. Further, the minute layer on the outer surface of the hollow fiber membrane makes it possible to increase a clogging-possible region of the membrane, which is effective to suppress lower the influence of clogging on the performance of the membrane. Furthermore, the intermediate layer with substantially no void indicates the membrane sectional structure having no void attributed to voids with diameters of 0.5 $\mu$m or more or a sponge structure, when observed with a scanning electron microscope of a magnification of 1,000.

In the present invention, a filament of the spinning solution discharged from the nozzle is allowed to pass through an uniform drying region of a low temperature. By doing so, initiation of phase separation of the filament proceeds, so that a minute layer is formed on the outermost layer of the hollow fiber membrane in the solidifying bath. For this reason, phase separation of a whole of the membrane is considered to mildly proceed, and the volume of pores is considered to be suppressed to be relatively small. Accordingly, there can be formed a structure suitable for formation of a protective layer capable of suppressing infiltration of a protein into the surface layers of the membrane, i.e., so-called clogging (see Fig. 1). However, pulling the hollow fiber membrane at an increased draft ratio during the formation of the membrane, or washing the hollow fiber membrane with a washing liquid which flows opposite the feeding of the membrane, immediately after the formation of the membrane, deforms the surface of the hollow fiber membrane to thereby destruct the uniform structure of the membrane suitable for formation of a protective layer. The protective layer is a barrier layer which is irreversibly formed by a protein in blood plasma. The protective layer shows higher resistance in the blood plasma than that in an aqueous solution, so that an overall mass transfer coefficient in the blood plasma, i.e., Ko$\beta$2, is smaller than that in the water system. However, the protective layer has an effect to suppress a change or variability in the performance of the membrane due to clogging. The foregoing is a hypothesis which should be made, also taken into consideration an influence of a pore distribution, etc. However, it is believed that the structure of the hollow fiber membrane will be close to an ideal one by combining the above-described various means, although the present technology is still insufficient to analyse it.

EXAMPLES

Hereinafter, the effectiveness of the present invention will be described by way of Examples, which, however, should not be construed as limiting the scope of the present invention in any way. The physical properties of the following Examples are evaluated by the methods described below.

1. Water Permeability

The circuit at the blood outlet portion of a dialyzer (on the side of the outlet rather than a pressure-measuring point) was pinched and sealed with a forceps. Pure water maintained at 37°C was poured into a pressurized tank, and the pure water was fed to the blood passage side of the dialyzer thermally insulated in a 37°C thermostatic tank, while a pressure being controlled with a regulator. Then, the amount of a filtrate flowing out of the dialyzing fluid side of the dialyzer was measured. A transmembrane pressure difference (TMP) was defined by the equation:

$$TMP = (Pi + Po)/2,$$

wherein Pi was a pressure on the inlet side of the dialyzer; and Po, a pressure on the outlet side of the dialyzer. The TMP was changed at 4 points, and filtration flow rates were measured. A water permeability (mL/hr./mmHg) was calculated from a slope of their relationship. The correlation coefficient of the TMP and the filtration flow rate should be 0.99 or more. To decrease an error in pressure loss due to the circuit, TMP was measured under a pressure of 100 mmHg or lower. The water permeability of a hollow fiber membrane was calculated from the membrane area and the water permeability of the dialyzer:

$$UFR(H) = UFR(D)/A,$$

wherein UFR(H) was the water permeability (mL/m$^2$/hr./mmHg) of the hollow fiber membrane; UFR(D) was the water permeability (mL/hr./mmHg) of the dialyzer; and A was the membrane area (m$^2$) of the dialyzer.

2. Calculation of Membrane Area

The membrane area of the dialyzer was determined based on the inner diameter of the hollow fiber membrane:

$$A = n \times \pi \times d \times L,$$

wherein n was the number of hollow fiber membranes in the dialyzer; $\pi$ was a ratio of the circumference of a circle to its diameter; d was the inner diameter (m) of a hollow fiber membrane; and L was the effective length (m) of the hollow fiber membranes in the dialyzer.

3. Overall Mass Transfer Coefficient

(1) Overall Mass Transfer Coefficient (Komyo) of Aqueous Myoglobin Solution

A dialyzing fluid which contained 0.01% of myoglobin (manufactured by Kishida Chemical Co., Ltd.) was allowed to flow into a blood purifier (membrane area (A'): 15,000 cm$^2$) primed and wetted with a physiological salt solution, as a single path, at a flow rate (Qbin) of 200 ml/min. on the blood side, without filtration thereof, while a dialyzing fluiud was allowed to flow at a flow rate (Qd) of 500 ml/min. on the dialyzing fluid side. The clearance (CLmyo, ml/min.) and the overall mass transfer coefficient (Komyo, cm.min.) of the blood purifier were calculated from the myoglobin concentration (Cbin) of the first myoglobin solution and the myoglobin concentration (Cbout) of the solution which had passed through and flowed out of the blood purifier. The measurement was conducted at 37°C.

$$CLmyo = (Cbin - Cbout)/Cbin \times Qbin$$

$$Komyo = Qbin/((A' \times (1 - Qbin/Qd)) \times$$

$$LN((1 - CL/Qd)/(1 - CL/Qb))$$

(2) Overall Mass Transfer Coefficient (Ko$\beta$2) of Blood Plasma Solution of $\beta$2-Microgloburin

($\beta$2-MG)

Blood plasma with a protein concentration of 6 to 7 g/dl was separated from ACD-added bovine blood by centrifugation. Blood plasma for use in a dialyzing test was admixed with heparin sodium (2,000 to 4,000 unit/L) and $\beta$2-microglobulin (a gene-recombination product manufactured by Wako Pure Chemical Industries, Ltd.) (about 0.01 mg/dl). Blood plasma for use in circulation was admixed with heparin sodium alone. At least 2 L of the blood plasma for use in circulation was prepared per one blood purifier. The blood plasma for use in circulation was allowed to flow into a blood purifier (membrane area (A'): 15,000 cm$^2$) primed and wetted with a dialyzing fluid, at a flow rate of 200 ml/min. At this moment of time, the dialyzing fluid side of the blood purifier was filled with a filtrate of the blood plasma which was being filtered at a Qf of 15 ml/min. After the filtrate had filled the dialyzing fluid side, the dialyzing fluid side was capped, so that the blood plasma was circulated only on the blood side of the blood purifier for one hour. After completion of the circulation, the blood plasma was changed over to the blood plasma for use in dialyzing test. This blood plasma was allowed to flow in a single path while being filtered so that Qbin could be 200 ml/min., and Qbout, 185 ml/min., meanwhile the dialyzing fluid was allowed to flow so that Qdin could be 500 ml/min. After 4 minutes had passed since the start of dialysis, the blood plasma Qbout on the blood side was sampled. The clearance (CL$\beta$2, ml/min.) and the overall mass transfer coefficient (Ko$\beta$2, cm/min.) of the blood purifier were calculated from the $\beta$2-MG concentration (Cbin) of the blood plasma solution, the $\beta$2-MG concentration (Cbout) of the same solution which had passed through the blood purifier and flowed out of the blood purifier, and the flow rate thereof. All the operations were conducted at 37°C.

$$CL\beta2 = (Cbin \times Qbin - Cbout \times Qbout)/Cbin$$

$$Ko\beta2 = Qbin/((A' \times (1 - Qbin/Qd)) \times$$
$$LN((1 - CL/Qd)/(1 - CL/Qb))$$

4. Retention

Two blood purifiers of the same type and the same lot (membrane areas of 1.5 m² based on the inner diameter of hollow fiber membranes) were prepared. The CLmyo of one of the blood purifiers was measured by the above-described method, and the CLβ2 of the other blood purifier was measured by the above-described method. After that, the blood purifier was washed with water for 5 minutes at the same flow rate as that for the measurement. The CLmyo of the washed blood purifier was measured, and a ratio of this CLmyo to the CLmyo of the first blood purifier was calculated. When quite no change was found in the performance of the blood purifier due to the blood circulation, the values of CLmyo of the two blood purifiers were equal to each other, and the retention was 100%.

$$Retention (\%) = CLmyo \text{ found after blood}$$
$$circulation/normal CLmyo \times 100$$

5. Porosity

A bundle of hollow fiber membranes immersed in pure water for one hour or longer was dewatered by centrifugation at 900 rpm for 5 minutes, and the weight of the bundle was measured. After that, the bundle was bone-dried in a drier, and the weight of the dried bundle was measured (Mp).

$$Wt \text{ (the weight of water in void pores)} =$$
$$\text{the weight of the bundle after the centrifugation}$$
$$- Mp$$

$$Volume \ porosity \ (Vt)\% =$$
$$Wt/(Wt + Mp/polymer \ density) \times 100$$

6. Yield Strength

TENSILON UTM II manufactured by Toyo Baldwin Co., Ltd. was used to measure a yield strength at a pulling rate of 100 mm/min. with a distance of 100 mm between each of chucks.

7. Unevenness in Thickness

The cross-sections of 100 hollow fiber membranes were observed with a projector of magnification of 200. One hollow fiber membrane having the largest difference in its thickness was selected from the hollow fiber membranes in one view field, and the cross section of this hollow fiber membrane was measured with respect to its thickest portion and its thinnest portion.

$$\text{Unevenness in thickness} =$$
$$\text{thinnest portion/thickest portion}$$

The thickness of a hollow fiber membrane was perfectly even when the Unevenness is one (1).

8. Calculation of Amount of Leaked Protein

Bovine blood admixed with citric acid to be inhibited from coagulating was adjusted to 25 to 30% in hemetocrit and to 6 to 7 g/dl in protein concentration. This bovine blood was fed to a blood purifier at a rate of 200 mL/min. and at 37°C to filter the bovine blood at a constant flow rate (Qf: ml/min.). The resulting filtrate was returned to the blood to thereby form a circulation system. A filtrate flow rate was measured at every 15 minute interval, and the filtrate from the blood purifier was collected. The concentration of protein in the filtrate was measured. The concentration of protein in blood plasma was measured using a kit for extracorporeal diagnosis (Micro TP-Test Wako manufactured by Wako Pure Chemical Industries, Ltd.). The average amount of leaked protein was determined based on the data recorded for 2 hours, from the following equation, and the amount of leaked protein (TPL) as a result of conversion in terms of 3L water removal was calculated.

$$\text{Integrated filtered amount (ml)} =$$
$$t_1(\text{min.}) \times C_{t1}(\text{ml/min.}) + (t_2 - t_1)(\text{min.}) \times C_{t2}(\text{ml/min.})$$
$$+ (t_3 - t_2)(\text{min.}) \times C_{t3}(\text{ml/min.}) \ldots (t_{120} - t_n)(\text{min.}) \times$$
$$C_{120\ \text{min.}}(\text{ml/min.})$$

t: measuring time (min.)
C: filtration flow rate (ml/min.)

$$\text{Concentration of protein in filtrate} =$$
$$a \times Ln\ (\text{integrated filtered amount}) + b$$

The values of a and b were determined from the concentration of the protein in the filtrate at each of the measuring points and Ln (the integrated filtered amount).

$$\text{TPL (average)} = -a + b + a \times Ln\ (\text{integrated filtered}$$
$$\text{amount} \times 2)$$

$$\text{TPL (converted in terms of 3L water removal) (g)} =$$
$$\text{TPL (average)} \times 30/1{,}000$$

Reproducibility of the blood performance and performance stability of the blood purifier were evaluated using the TPL value as a result of conversion in terms of 3 L water removal, as an indication.

9. Measurement of Inner and Outer Diameters and Thickness of Hollow Fiber Membrane

Samples of the sections of hollow fiber membranes were obtained as follows. Prior to observation and measurement, preferably, hollow fiber membranes were washed to remove a hollow portion-forming material therefrom and were then dried. While the drying method is not limited, hollow fiber membranes, if remarkably deformed by drying, preferably should be washed to remove the hollow portion-forming material, and be then perfectly displaced with pure water and be observed in wet states. Such a proper number of hollow fiber membranes as were not slipped down from a hole of φ3 mm opened at the center of a slide glass were threaded through this hole and were cut on the upper and lower surfaces of the slide glass, with a razor, to obtain samples of the sections of the hollow fiber membranes. After that, a projector, Nikon-V-12A, was used to measure the minor axes and major axes of the sections of the hollow fiber membranes. In concrete, one section of the hollow fiber membrane was measured with respect its minor axes and major axes each in two directions; and the respective arithmetic average values were defined as the inner diameter and the outer diameter of the section of the one hollow fiber membrane. The thickness of the hollow fiber membrane was calculated by the equation: (the outer diameter - the inner diameter)/2. The five sections of the hollow fiber membranes were measured by the same method as described above to find the respective average values as the inner diameter and thickness.

10. Measurement of Pore Volume Porosity and Average Pore Radius of Hollow Fiber Membrane

Ten hollow fiber membranes sufficiently wetted with pure water were cut into pieces with lengths of about 5 mm, from which excessive water was removed with filter paper. Such pieces of the hollow fiber membranes were packed in a sealed pan so as to measure the melting curve thereof, using a differential scanning calorimeter (DSC-7 or Pyris 1, manufactured by Perkin-Elmer). The measurement was conducted at a temperature-raising rate of 2.5°C/min. within a temperature range of -45 to 15°C. The water in the pores of the membrane was affected by the base material of the membrane and showed depression of freezing point, thus showing a peak at a region different from the region of free water (which melts at and around 0°C), i.e., at a temperature region lower than that of free water. A quantity of heat of melting ($\Delta Hp$) of a region enclosed by the peak and the base line of the portion of water showing depression of freezing point was determined. Then, the amount of water in the pores (Wp) was calculated from the quantity of heat of melting ($\Delta Hm$) per unit weight of water. The sample measured with DSC was bone-dried, and the weight of evaporated water (total moisture weight Wt) was measured. The pore volume porosity (Vp) was calculated from these values by the following equations:

$$Wp = \Delta Hp / \Delta Hm$$

$$Vp\ (\%) = Wp / (Wt + Mp/\rho p) \times 100$$

Mp: the weight of a polymer = the weight of a sample - the total moisture amount (Wt)
pp: a specific gravity of the polymer

A peak top of the peak of the portion of water showing depression of freezing point was read from the melting curve obtained as above. The radius (r) of the pore could be simply calculated by the following equation, from the degree of depression of freezing point (ice point) attributed to capillary condensation of water in the pore. In the present invention, the average radius of the pores was defined as a value found by this measuring method.

$$r(\text{Å}) = \text{degree of depression of ice point } (^{\circ}C)/164$$

(Example 1)

Cellulose triacetate (manufactured by DAICEL CHEMICAL INDUSTRIES, LTD.) (19% by mass), N-methyl-2-pyrrolidone (NMP, manufactured by Mitsubishi Chemical Corporation) (56.7% by mass) and triethylene glycol (TEG, manufactured by MITSUI CHEMICALS, INC.) (24.3% by mass) were heated and homogeneously melted to form a membrane-forming solution, which was then defoamed. The resultant membrane-forming solution was allowed to sequentially pass through a two-staged sintered filter of 10 μm and 5 μm, and was then discharged from a tube-in-orifice nozzle heated to 102°C, together with previously deaired liquid paraffin as a hollow portion-forming material. The resulting semi-solid hollow fiber membrane was allowed to pass through a 70 mm drying section regulated at 12°C, sealed from an external air by a spinning tube, and was then solidified in an aqueous 20% by mass NMP/TEG (7/3) solution of 40°C, undergoing a water-washing bath of 30°C, followed by a 60% by mass glycerin bath of 50°C. The resulting hollow fiber membrane was then dried in a drier and was wound up at a spinning rate of 30 m/min. The draft ratio of the membrane-forming solution was 7. The difference between the maximum value and the minimum value of the nozzle slit width was 7 $\mu$m. A ceramic heat-insulating material with a thickness of 5 mm was inserted between the nozzle block and the spinning tube. The water washing bath was inclined an angle of 2.5° so that washing water could slowly flow down, in parallel to the hollow fiber membrane in the same direction. The water washing bath was five-staged. The draw ratio of the hollow fiber membrane in the entire water washing bath was 1.0001. The draw ratio of the hollow fiber membrane found within the region from the outlet of the solidifying bath to the winding site was 1.04.

The inner diameter of the resultant hollow fiber membrane was 200.5 $\mu$m; the thickness thereof was 15.8 $\mu$m; the unevenness in thickness was 0.7; the porosity thereof was 75.8%; the yield strength was 12.5 g; and the average pore radius thereof was 180 angstrom (see Table 1). The structure of the section of this hollow fiber membrane was observed with FE-SEM (with a magnification of 5,000), with the result that a minute layer with a thickness of about 0.1 $\mu$m was observed on the outer surface of the membrane.

A blood purifier having a membrane area of 1.5 m$^2$ was assembled, using the resultant hollow fiber membrane. The effective length of the hollow fiber membrane packed in a module was 22.5 cm. This blood purifier was measured in its water permeability, overall mass transfer coefficient ratio (Ko$\beta$2/Komyo) and performance retention. Protein-leaking tests were conducted on 5 modules so as to evaluate the blood performance. The results are shown in Table 2. The $\beta$2-MG-removing performance expected for the modules was as high as average 61.7 as CL$\beta$2, and variability in the performance was small. The modules showed high performance retention and showed high reproducibility in the protein-leaking tests, and the amounts of leaked proteins were suppressed to be small.

(Example 2)

Cellulose triacetate (manufactured by DAICEL CHEMICAL INDUSTRIES, LTD.) (18% by mass), NMP (57.4% by mass) and TEG (24.6% by mass) were homogeneously melted to form a membrane-forming solution, which was then defoamed. The resultant membrane-forming solution was allowed to sequentially pass through a two-staged sintered filter of 10 $\mu$m and 5 $\mu$m, and was then discharged from a tube-in-orifice nozzle heated to 105°C, together with previously deaired liquid paraffin as a hollow portion-forming material. The resulting semi-solid hollow fiber membrane was allowed to pass through a 50 mm drying section under a homogeneous atmosphere regulated to 5°C, sealed from an external air by a spinning tube, and was then solidified in an aqueous 20% by mass NMP/TEG (7/3) solution of 40°C, undergoing a water-washing bath of 30°C, followed by a 60% by mass glycerin bath of 50°C. The resulting hollow fiber membrane was then dried in a drier and was wound up at a spinning rate of 85 m/min. The draft ratio of the membrane-forming solution was 7. The difference between the maximum value and the minimum value of the nozzle slit width was 8 $\mu$m. A ceramic heat-insulating material with a thickness of 8 mm was inserted between the nozzle block and the spinning tube. The water washing bath was inclined an angle of 1° so that the hollow fiber membrane could slowly flow down, in parallel to the washing water in the same direction. The water washing bath was seven-staged. The draw ratio of the hollow fiber membrane in the entire water washing bath was 1.005. The draw ratio of the hollow fiber membrane found within the region from the outlet of the solidifying bath to the winding site was 1.03.

The inner diameter of the resultant hollow fiber membrane was 199.8 $\mu$m; the thickness thereof was 15.4 $\mu$m; the unevenness in thickness was 0.8; the porosity thereof was 78.5%; the yield strength was 12.3 g; and the average pore radius thereof was 260 angstrom (see Table 1). The structure of the section of this hollow fiber membrane was observed with FE-SEM (with a magnification of 5,000), with the result that a minute layer with a thickness of about 0.1 $\mu$m was observed on the outer surface of the membrane.

The same evaluations as in Example 1 were made on the resultant hollow fiber membrane. The results are shown in Table 2. The $\beta$2-MG-removing performance expected for the modules was as high as average 68.7 as CL$\beta$2, and variability in the performance was small. The modules showed high performance retention and showed high reproducibility in the protein-leaking tests, and the amounts of leaked proteins were suppressed to be small.

(Example 3)

Polyether sulfone (highly polymerized polyether sulfone 7300P, manufactured by Sumitomo Chemical Company, Limited) (23% by mass), polyvinyl pyrrolidone (PVP K-90, manufactured by BASF) (2% by mass), N-methyl-2-pyrrolidone (NMP, manufactured by Mitsubishi Chemical Corporation) (45% by mass) and polyethylene glycol (PEG 200, manufactured by DAI-ICHI KOGYO SEIYAKU CO., LTD.) (30% by mass) were homogeneously melted to form a membrane-forming solution, which was then defoamed. The resultant membrane-forming solution was allowed to sequentially pass through a two-staged sintered filter of 10 $\mu$m and 5 $\mu$m, and was then discharged from a tube-in-orifice nozzle heated to 128°C, together with a nitrogen gas as a hollow portion-forming material. The resulting semi-solid hollow fiber membrane was allowed to pass through a 8 mm drying section regulated at 10°C, sealed from an external air by a spinning tube, and was then solidified in an aqueous 40% by mass NMP/PEG 200 (6/4) solution of 40°C, undergoing a water-washing bath of 50°C, followed by a 60% by mass glycerin bath of 50°C. The resulting hollow fiber membrane was then dried in a drier and was wound up at a spinning rate of 70 m/min. The draft ratio of the membrane-forming solution was 4.8. The difference between the maximum value and the minimum value of the nozzle slit width was 7 $\mu$m. The water washing bath was inclined an angle of 2.5° so that washing water could slowly flow down in parallel to the hollow fiber membrane in the same direction. The water washing bath was five-staged. The draw ratio of the hollow fiber membrane in the entire water washing bath was 1.001. The draw ratio of the hollow fiber membrane found within the region from the outlet of the solidifying bath to the winding site was 1.03.

The inner diameter of the resultant hollow fiber membrane was 200 $\mu$m; the thickness thereof was 29.8 $\mu$m; the unevenness in thickness was 0.7; the porosity thereof was 74.8%; the yield strength was 23.5 g; and the average pore radius thereof was 160 angstrom (see Table 1). The structure of the section of this hollow fiber membrane was observed with FE-SEM (with a magnification of 5,000), with the result that a minute layer with a thickness of about 0.1 $\mu$m was observed on the outer surface of the membrane.

A blood purifier having a membrane area of 1.5 m$^2$ was assembled, using the resultant hollow fiber membrane. The effective length of the hollow fiber membrane packed in a module was 22.5 cm. This blood purifier was measured in its water permeability, overall mass transfer coefficient ratio (Ko$\beta$2/Komyo) and performance retention. Protein-leaking tests were conducted on 5 modules so as to evaluate the blood performance. The results are shown in Table 2. The $\beta$2-MG-removing performance expected for the modules was as high as average 58.7 as CL$\beta$2, and variability in the performance was small. The modules showed high performance retention and showed high reproducibility in the protein-leaking tests, and the amounts of leaked proteins were suppressed to be small.

(Comparative Example 1)

Cellulose triacetate (manufactured by DAICEL CHEMICAL INDUSTRIES, LTD.) (19% by mass), NMP (56.7% by mass) and TEG (24.3% by mass) were homogeneously melted to form a membrane-forming solution, which was then defoamed. The resultant membrane-forming solution was allowed to sequentially pass through a two-staged sintered filter of 20 $\mu$m and 20 $\mu$m, and was then discharged from a tube-in-orifice nozzle heated to 105°C, together with previously deaired liquid paraffin as a hollow portion-forming material. The resulting semi--solid hollow fiber membrane was allowed to pass through a 70 mm drying section under a homogeneous atmosphere regulated to 12°C, sealed from an external air by a spinning tube, and was then solidified in an aqueous 20% by mass NMP/TEG (7/3) solution of 40°C, undergoing a water-washing bath of 30°C, followed by a 60% by mass glycerin bath of 50°C. The resulting hollow fiber membrane was then dried in a drier and was wound up at a spinning rate of 85 m/min. The draft ratio of the membrane-forming solution was 11. The nozzle block was in direct contact with the enclosure of the aeration feeding region. The difference between the maximum value and the minimum value of the nozzle slit width was 7 $\mu$m. The water washing bath was inclined an angle of 0.5° so that the hollow fiber membrane could be gently inclined upward, and the washing water and the hollow fiber membrane were allowed to flow in the opposite directions to each other. The water washing bath was seven-staged. The draw ratio of the hollow fiber membrane in the entire water washing bath was 1.12. The draw ratio of the hollow fiber membrane found within the region from the outlet of the solidifying bath to the winding site was 1.2.

The inner diameter of the resultant hollow fiber membrane was 199.8 $\mu$m; the thickness thereof was 15.0 $\mu$m; the unevenness in thickness was 0.6; the porosity thereof was 82.3%; the yield strength was 12.6 g; and the average pore radius thereof was 150 angstrom (see Table 1). The structure of the section of this hollow fiber membrane was observed with FE-SEM (with a magnification of 5,000), with the result that no minute layer was observed on the outer surface of the membrane.

The same evaluations as in Example 1 were made on the resultant hollow fiber membrane. The results are shown in Table 2. The $\beta$2-MG-removing performance expected for the modules was as high as average 60 as CL$\beta$2, but was largely variable within a range of from 52 to 65. The modules showed low performance retention and also showed low reproducibility in the protein-leaking tests.

(Comparative Example 2)

Cellulose triacetate (manufactured by DAICEL CHEMICAL INDUSTRIES, LTD.) (17.5% by mass), NMP (57.75% by mass) and TEG (24.75% by mass) were homogeneously melted to form a membrane-forming solution, which was then defoamed. The resultant membrane-forming solution was allowed to sequentially pass through a two-staged sintered filter of 15 $\mu$m and 15 $\mu$m, and was then discharged from a tube-in-orifice nozzle heated to 105°C, together with previously deaired liquid paraffin as a hollow portion-forming material. The resulting semi-solid hollow fiber membrane was allowed to pass through a 50 mm drying section under a homogeneous atmosphere regulated to 30°C, sealed from an external air by a spinning tube, and was then solidified in an aqueous 20% by mass NMP/TEG (7/3) solution of 40°C, undergoing a water-washing bath of 30°C, followed by a 60% by mass glycerin bath of 50°C. The resulting hollow fiber membrane was then dried in a drier and was wound up at a spinning rate of 30 m/min. The draft ratio of the membrane-forming solution was 11. The nozzle block was in direct contact with the enclosure of the aeration feeding region. The difference between the maximum value and the minimum value of the nozzle slit width was 10 μm. The water washing bath was inclined an angle of 3° so that the hollow fiber membrane could be mildly inclined downward, and the washing water and the hollow fiber membrane were allowed to flow in parallel to each other in the same direction. The water washing bath was five-staged. The draw ratio of the hollow fiber membrane in the entire water washing bath was 1.2. The draw ratio of the hollow fiber membrane found within the region from the outlet of the solidifying bath to the winding site was 1.3.

The inner diameter of the resultant hollow fiber membrane was 198.5 μm; the thickness thereof was 14.7 μm; the unevenness in thickness was 0.7; the porosity thereof was 81.4%; the yield strength was 7.9 g; and the average pore radius thereof was 320 angstrom (see Table 1). The structure of the section of this hollow fiber membrane was observed with FE-SEM (with a magnification of 5,000), with the result that no minute layer was observed on the outer surface of the membrane.

The same evaluations as in Example 1 were made on the resultant hollow fiber membrane. The results are shown in Table 2. The filament strength was low, since the temperature of the aeration feeding region through which the hollow fiber membrane discharged from the nozzle was allowed to pass was high, in the spinning step. The β2-MG-removing performance expected for the modules was as high as average 72 as CLβ2, but was largely variable within a range of from 65 to 79. The modules showed low performance retention and also showed low reproducibility in the protein-leaking tests. Further, the radius of the pores of the membrane was large, and thus, the amount of leaked protein was large.

(Comparative Example 3)

Cellulose triacetate (manufactured by DAICEL CHEMICAL INDUSTRIES, LTD.) (19% by mass), NMP (56.7% by mass) and TEG (24.3% by mass) were homogeneously melted to form a membrane-forming solution, which was then defoamed. The resultant membrane-forming solution was allowed to sequentially pass through a two-staged sintered filter of 15 μm and 15 μm, and was then discharged from a tube-in-orifice nozzle heated to 105°C, together with previously deaired liquid paraffin as a hollow portion-forming material. The resulting semi-solid hollow fiber membrane was allowed to pass through a 50 mm drying section under a homogeneous atmosphere regulated to 30°C, sealed from an external air by a spinning tube, and was then solidified in an aqueous 30% by mass NMP/TEG (7/3) solution of 50°C, undergoing a water-washing bath of 30°C, followed by a 65% by mass glycerin bath of 55°C. The resulting hollow fiber membrane was then dried in a drier and was wound up at a spinning rate of 75 m/min. The draft ratio of the membrane-forming solution was 11. The nozzle block was in direct contact with the enclosure of the aeration feeding region. The difference between the maximum value and the minimum value of the nozzle slit width was 10 μm. The water washing bath was inclined an angle of 3° so that the hollow fiber membrane could be gently inclined upward, and the washing water and the hollow fiber membrane were allowed to flow in the opposite directions to each other. The water washing bath was five-staged. The draw ratio of the hollow fiber membrane in the entire water washing bath was 1.14. The draw ratio of the hollow fiber membrane found within the region from the outlet of the solidifying bath to the winding site was 1.2.

The inner diameter of the resultant hollow fiber membrane was 199.2 μm; the thickness thereof was 15.8 μm; the unevenness in thickness was 0.7; the porosity thereof was 85.6%; the yield strength was 8.5 g; and the average pore radius thereof was 350 angstrom (see Table 1). The structure of the section of this hollow fiber membrane was observed with FE-SEM (with a magnification of 5,000), with the result that no minute layer was observed on the outer surface of the membrane.

The same evaluations as in Example 1 were made on the resultant hollow fiber membrane. The results are shown in Table 2. The filament strength was slightly low, since the temperature of the aeration feeding region through which the hollow fiber membrane discharged from the nozzle was allowed to pass was high, in the spinning step. The β2-MG-removing performance expected for the modules was as high as average 56.7 as CLβ2, but was largely variable within a range of from 52 to 63. The modules showed low performance retention and also showed low reproducibility in the protein-leaking tests. Further, the radius of the pores of the membrane was large, and thus, the amount of leaked protein was large.

[Table 1]

|  | Unevenness in thickness | Porosity (%) | Yield strength (g) | Av. value of pore radius (Å) | Vp (%) |
|---|---|---|---|---|---|
| Ex. 1 | 0.7 | 75.8 | 12.5 | 180 | 44 |
| Ex. 2 | 0.8 | 78.5 | 12.3 | 260 | 38 |
| Ex. 3 | 0.7 | 74.8 | 23.5 | 160 | 42 |

(continued)

| | Unevenness in thickness | Porosity (%) | Yield strength (g) | Av. value of pore radius (Å) | Vp (%) |
|---|---|---|---|---|---|
| C. Ex. 1 | 0.6 | 82.3 | 12.6 | 150 | 52 |
| C. Ex. 2 | 0.7 | 81.4 | 7.9 | 320 | 57 |
| C. Ex. 3 | 0.7 | 85.6 | 8.5 | 350 | 48 |

[Table 2]

| | UFR (ml/hr./mmHg/m$^2$) | Koβ2/Komyo CLβ/CLmyo | Performance retention (%) | TPL (g) |
|---|---|---|---|---|
| Ex. 1 | 243 | 0.85 62,62,61/71,70,69 | 71 | 0.5,0.6,0.6,0.7,0.8 |
| Ex. 2 | 268 | 0.93 69,69,68/72,72,73 | 68 | 0.8,0.9,0.9,1.1,1.2 |
| Ex. 3 | 168 | 0.82 59,59,58/69,69,68 | 68 | 1.0,1.0,1.1,1.1,1.2 |
| C. Ex. 1 | 264 | 1.09 65,63,52/68,68,68 | 53 | 0.8,0.9,1.2,1.5,1.5 |
| C. Ex. 2 | 335 | 1.08 79,72,65/69,68,67 | 59 | 1.2,1.3,1.5,1.7,2.0 |
| C. Ex. 3 | 224 | 1.04 63,55,52/57,57,57 | 40 | 1.4,1.7,2.0,2.2,2.5 |

INDUSTRIAL APPLICABILITY

The hollow fiber type blood purifiers according to the present invention have higher water permeability, and have stability in blood performance by keeping the performance in blood and the performance in aqueous solutions under constant conditions. Consequently, the blood purifiers show less variability in performance, independently of patients' body conditions, and thus are expected to exhibit constant treating effects. Therefore, the present invention will contribute much to the development of this industrial field.

**Claims**

1. A hollow fiber membrane excellent in performance stability, which has an average thickness of from 10 to 50 μm and an average pore radius of from 150 to 300 Å, and which shows a pure water permeability of 150 to 1,500 mL/m$^2$/hr./mmHg at 37°C, **characterized in that** the ratio of the overall mass transfer coefficient (Koβ2) of a blood plasma solution of β2-microgloburin to the overall mass transfer coefficient (Komyo) of an aqueous myoglobin solution (i.e., Koβ2/Komyo) is from 0.7 to 1.0.

2. The hollow fiber membrane of claim 1, wherein the pore volume porosity is from 10 to 50%.

3. The hollow fiber membrane of claim 1 or 2, wherein minute layers are formed on the inner and outer surfaces of the hollow fiber membrane, and an intermediate layer between these minute layers is a support layer having substantially no void.

4. A blood purifier hardly clogged and excellent in performance stability, which is assembled using the hollow fiber membrane defined in any one of claims 1 to 3, wherein a myoglobin clearance measured after circulation of blood plasma on the blood passage side of the blood purifier for one hour is 60% or more of a myoglobin clearance measured before the circulation of the blood plasma.

**5.** The blood purifier of claim 4, wherein variability in $\beta$2-microglobulin clearance measured after the circulation of the blood plasma for one hour is 8% or less.

**6.** A process for manufacturing the hollow fiber membrane defined in any one of claims 1 to 3, by way of a dry-wet type spinning method, **characterized in that** a spinning dope is discharged from a nozzle to form a semi-solid filament hollow inside, which is then immersed in a solidifying bath to be solidified to form a hollow fiber membrane, which is sequentially washed in a water washing tank, while the hollow fiber membrane and a washing liquid are being fed in the same direction.

[Fig. 1]

【Fig. 2】

【Fig. 3】

**EP 2 005 983 A2**

**REFERENCES CITED IN THE DESCRIPTION**

**Patent documents cited in the description**

- JP 101277631998 A
- JP 101657741998 A
- JP 20001531342000 A
- JP 102164891998 A
- JP 101089071998 A
- JP 20003009732000 A
- JP 6642905 B
- JP 8008970 A